# EUROPEAN PATENT APPLICATION

(11) **EP 2 372 727 A1**
(43) Date of publication of application: **05.10.2011**
(21) Application number: 09834554.9
(22) Date of filing: 21.04.2009
(51) Int. Cl.: H01F 27/00, G01N 30/88, H01F 41/00

(54) **METHOD FOR PREDICTING THE PROBABILITY OF ABNORMALITY OCCURRENCE IN OIL-FILLED ELECTRICAL APPARATUS**

(30) Priority: 25.12.2008 JP 2008330751
(71) Applicant: Mitsubishi Electric Corporation, Tokyo 100-8310 (JP)
(72) Inventor: TOYAMA, Satoru, Tokyo 100-8310 (JP); MIZUNO, Kota, Tokyo 100-8310 (JP); TANIMURA, Junji, Tokyo 100-8310 (JP); AMIMOTO, Tsuyoshi, Tokyo 100-8310 (JP)
(74) Representative: Popp, Eugen
(86) International application number: PCT/JP2009/057890
(87) International publication number: WO 2010/073748

(57) **Abstract**

A method for predicting the possibility of occurrence of an anomaly includes compositionally analyzing an insulating oil collected from a transformer in operation, calculating an estimated value of a concentration of dibenzyl disulfide in the insulating oil in its virgin state from the compositional analysis, and predicting the possibility of occurrence of anomaly in the oil-filled electrical apparatus from the level of the estimated value.

## Description

### TECHNICAL FIELD

The present invention relates to a method for predicting the possibility of occurrence of an anomaly about sulfidation corrosion in an oil-filled electrical apparatus such as a transformer. More specifically, the present invention relates to a method for predicting the possibility of occurrence of an anomaly resulting from sulfidation corrosion, based on the concentration in insulating oil of a reaction product between a sulfur based compound and copper and responsible for sulfidation corrosion.

### BACKGROUND ART

There are some oil-filled electrical apparatuses such as an oil-filled transformer which may include an insulating oil containing a sulfur component. In this case, it is known that a reaction between a copper part of the oil-filled electrical apparatus and the sulfur component in the insulating oil produces electrically-conductive copper sulfide (sulfidation corrosion), which adheres to insulating paper or the like and causes insulation breakdown, which may result in fatal damage to the oil-filled electrical apparatus. However, the details of the mechanism for copper sulfide production are not known. Furthermore, it is difficult to diagnose whether or not an existing oil-filled electrical apparatus has an anomaly resulting from production of copper sulfide, without stopping the apparatus. Accordingly, in order to avoid various problems arising from sulfidation corrosion, one currently has to depend on the development of an oil-filled electric apparatus with as little as possible tendency to experience occurrence of sulfidation corrosion.

Examples of the development of oil-filled electric equipment with less tendency to experience sulfidation corrosion include selecting an insulating oil less likely to cause sulfidation corrosion and developing technology for removing sulfur compounds from an insulating oil. The former is exemplified in Patent Document 1 which discloses a method for diagnosing sulfidation corrosiveness of an insulating oil. In the method, a certain amount of insulating oil and a copper plate having a predetermined surface area are enclosed within a container and heated at a predetermined temperature for a predetermined time period. Subsequently, the contents of the oil-dissolved copper and the sulfate ions contained in the insulating oil are measured to obtain the sum of contents, based on which a diagnosis is made (Patent Document 1: Japanese Patent Laying-Open No. 7-335446 (page 3, paragraph [0003]).

Meanwhile, as for occurrence of an oil-filled electrical apparatus failures other than sulfidation corrosion, there have been proposed numerous methods for diagnosing an overheat anomaly or an electric discharge anomaly by detecting a certain type of compound. For example, Patent Document 2 discloses a method for diagnosing an overheat anomaly or an electric discharge anomaly within an oil-filled electrical apparatus based on whether or not there is detection of acetic acid [CH₃COOH], 3-pentanone [CH₃CH₂COCH₂CH₃], 2.5-dimethylfuran [C₆H₈O], butylaldehyde [CH₃CHCHCHO], 2-methoxyethanol [C₃H₈O₂], methanethiol [CH₃SH], dimethyl sulfide [(CH₃)S₂], ammonia [NH₃], 1.3-diazine [C₄H₄N₂], methyl vinylacetylene [C₅H₆], and 2-methyl-1.3-butadiene [C₅H₈] which are compounds not usually detected from an oil-filled electrical apparatus during normal operation, but tend to be detected only when an overheat or electric discharge anomaly occurs (Patent Document 2: Japanese Patent Laying-Open No. 9-72892 (claim 2)).

Further, Patent Document 3 discloses a method using compounds in an electrically insulating oil to be described below for diagnosing an anomaly in an oil-filled electrical apparatus such as an oil-filled transformer, a reactor and an automatic regulator, an oil-filled cable and the like. In the disclosed method, based on each amount of various gases such as carbon dioxide [CO₂], carbon monoxide [CO], methane [CH₄], hydrogen [H₂], ethane [C₂H₆], ethylene [C₂H₄], and acetylene [C₂N₂] contained in an electric insulating oil of an oil-filled electrical apparatus such as an oil-filled transformer, a reactor and an automatic regulator, an oil-filled cable and the like, an estimation of degradation conditions and a diagnosis of an anomaly of the oil-filled electrical apparatus and the like are made (Patent Document 3: Japanese Patent Laying-Open No. 2000-241401 (page 2, paragraph [0006])).

Still further, Patent Documents 4 and 5 each disclose a method for diagnosing an anomaly in an insulating member of an oil-filled electrical apparatus, in which the amounts of hydroxymethylfurfural and furfural in the oil are quantified for use as indices of deterioration of insulating paper (Patent Document 4: Japanese Patent Laying-Open No. 5-315147 (page 2, claims 1 to 3), Patent Document 5: Japanese Patent Laying-Open No. 8-124751 (page 2, claim 2)).

However, although such methods for diagnosing an oil-filled electrical apparatus are effective for a diagnosis of current defective conditions of an oil-filled electrical apparatus, such as degraded insulating paper and flow electrification, these methods do not allow for a diagnosis of the possibility of future defective conditions of the oil-filled electrical apparatus resulting from production of copper sulfide.

### PRIOR ART DOCUMENTS

### PATENT DOCUMENTS

Patent Document 1: Japanese Patent Laying-Open No. 7-335446
Patent Document 2: Japanese Patent Laying-Open No. 9-72892
Patent Document 3 : Japanese Patent Laying-Open No. 2000-241401
Patent Document 4: Japanese Patent Laying-Open No. 5-315147
Patent Document 5: Japanese Patent Laying-Open No. 8-124751

### SUMMARY OF THE INVENTION

### PROBLEMS TO BE SOLVED BY THE INVENTION

An object of the present invention is to provide a method for predicting the possibility of occurrence of future defective conditions of an oil-filled electrical apparatus resulting from production of copper sulfide, by an analysis of the oil-filled electrical apparatus in the current state.

### MEANS FOR SOLVING THE PROBLEMS

As a result of diligent study, the present inventors have found a method in which the initial concentration of a substance responsible for production of copper sulfide contained in an insulating oil is estimated from an analysis of the oil degraded by aging, and the possibility of occurrence of defective conditions of an oil-filled electrical apparatus resulting from production of copper sulfide can be predicted from the estimated value of the initial concentration of the responsible substance.

That is, the present invention is a prediction method for predicting the possibility of occurrence of anomaly in an oil-filled electrical apparatus. The method includes compositionally analyzing an insulating oil collected from a transformer in operation, calculating an estimated value of a concentration of dibenzyl disulfide (hereinafter may be referred to as DBDS) in the insulating oil in its virgin state from the compositional analysis, and predicting the possibility of occurrence of anomaly in an oil-filled electrical apparatus from a level of the estimated value.

Preferably, in the present invention, concentrations of bibenzyl (hereinafter may be referred to as BiBZ) and/or dibenzyl sulfide (hereinafter may be referred to as DBS) and dibenzyl disulfide in the insulating oil are quantified by the compositional analysis of the insulating oil.

Further preferably, in the present invention, an estimated value of a decrement of the concentration of dibenzyl disulfide in the insulating oil relative to its virgin state is calculated from the concentration of bibenzyl and/or dibenzyl sulfide in the insulating oil quantified by the compositional analysis of the insulating oil,
the estimated value of the decrement is added to the concentration of dibenzyl disulfide in the insulating oil quantified by the compositional analysis, and
the estimated value of the concentration of dibenzyl disulfide in the insulating oil in its virgin state is calculated by the addition.

More preferably, in that case, a relational expression between a decrement of dibenzyl disulfide and an amount of produced bibenzyl and/or dibenzyl sulfide at each temperature is created in advance by quantifying, with varied elapsed time periods and temperature conditions, a decrement of dibenzyl disulfide and an amount of produced bibenzyl and/or dibenzyl sulfide in an insulating oil to which dibenzyl disulfide has been added and which is after expiration of a predetermined time period, and
the estimated value of the decrement of a concentration of dibenzyl disulfide in the insulating oil relative to its virgin state is calculated based on the relational expression and from a temperature condition of the insulating oil and the concentration of bibenzyl and/or dibenzyl sulfide in the insulating oil quantified by the compositional analysis.

Further preferably, quantification of the concentration of dibenzyl disulfide in the insulating oil and a quantification of the concentration or bibenzyl and/or dibenzyl sulfide in the insulating oil is achieved using gas chromatography.

### EFFECTS OF THE INVENTION

According to the present invention, the amount of copper sulfide to be produced due to age-related degradation of an oil-filled electrical apparatus can be predicted, and from this prediction, possibility of occurrence of an anomaly resulting from sulfidation corrosion of the aged oil-filled electrical apparatus can be predicted.

### BRIEF DESCRIPTION OF THE DRAWINGS

Fig. 1 is a graph showing a relationship between an amount of produced bibenzyl and a decrement of DBDS when heating was performed at 165°C.
Fig. 2 is a graph showing a relationship between an amount of produced dibenzyl sulfide and a decrement of DBDS when heating was performed at 165°C.
Fig. 3 is a graph showing a relationship between the temperature and a slope of a plotted relationship between the amount of bibenzyl and the decrement of DBDS.
Fig. 4 is a graph showing a relationship between the temperature and a slope of a plotted relationship between the amount of dibenzyl sulfide and the decrement of DBDS.

### MODES FOR CARRYING OUT THE INVENTION

Although it has been known that addition of DBDS to an insulating oil easily causes production of copper sulfide, the details of the mechanism for copper sulfide production had not been known. A diligent research of the reaction mechanism reveled that a reaction in which absorption of DBDS to a copper plate occurs as a first step, a reaction in which DBDS reacts with copper to produce a DBDS-Cu complex occurs as a second step, and a reaction in which a dibenzyl disulfide-Cu complex decomposes into a benzyl radical and a benzyl sulfenyl radical and copper sulfide occurs as a third step.

Further, a benzyl radical and a benzyl sulfenyl radical react with another benzyl radical and another benzyl sulfenyl radical, respectively, or react with each other to produce bibenzyl, dibenzyl sulfide and DBDS. Thus, quantification of bibenzyl and dibenzyl sulfide can provide an estimation of the consumed amount (decrement) of DBDS. Then, from the sum of the thus obtained estimated value of decrement of DBDS and the residual amount of DBDS obtained by analysis, the initial DBDS concentration can be estimated. Quantification of bibenzyl and dibenzyl sulfide can be achieved by various publicly known analysis methods, for example, gas chromatography.

In order to predict the possibility of occurrence of an anomaly due to sulfidation corrosion from the initial DBDS concentration in an insulating oil, for example, a corrosion test is carried out in advance using insulating oils having different initial DBDS concentrations, and a threshold value of the DBDS concentration is obtained from the test result. If the estimated value of the initial DBDS concentration is higher than the threshold value, then it can be predicted that the future occurrence of an anomaly due to sulfidation corrosion is highly probable. If the estimated value is lower than the threshold value, then it can be predicted that the future occurrence of an anomaly due to sulfidation corrosion is less probable.

An insulating oil contained in an oil-filled electrical apparatus to which the present invention is applied is, for example, a mineral oil or a synthetic oil containing a sulfur component, and preferably, an insulating oil containing a sulfur component.

### First Embodiment

The procedures of the present invention will be hereinafter described by way of specific study results.

First, in accordance with ASTM D 1275B, which is a test method of the ASTM (American Society for Testing and Materials), a transformer oil verified as free of corrosive sulfur is prepared. A predetermined amount of DBDS is then added to the transformer oil. In the present experiment, DBDS was added in the amounts of 30 and 300 ppm. Four grams of the transformer oil and a copper plate are enclosed in a jar having a capacity of 10 cc and then heated at a predetermined temperature for a predetermined time period after closing the jar with a rubber stopper.

DBDS, dibenzyl sulfide and bibenzyl contained in the heated transformer oil can be analyzed with a gas chromatograph/mass spectrograph (GC/MS) to obtain the concentration of these compounds from the analysis.

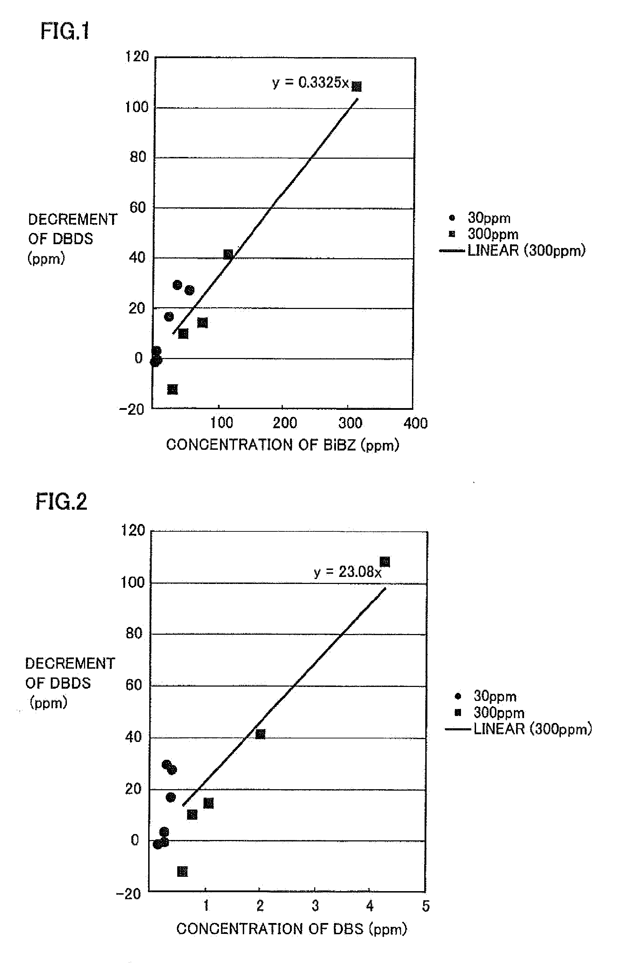

For example, Fig. 1 shows a relationship between the concentration of produced bibenzyl (in ppm) (i.e., the amount of produced BiBZ) and the decrement of DBDS concentration (in ppm) upon the expiration of different time periods when heating was performed at 165°C. Fig. 1 shows data sets of cases where the initial DBDS concentrations were 30 ppm and 300 ppm, respectively. In the drawing, " linear (300 ppm) " indicates a straight line fitted by the method of least square to the data set of the case where the initial DBDS concentration was 300 ppm, the line being represented by the following relational expression: y = 0.3325x, where y is a decrement of DBDS and x is a BiBZ concentration. As shown in Fig. 1, the amount of bibenzyl and the decrement of DBDS are well correlated. Thus, from their relational expression created from the data sets of Fig. 1 and the quantitative value of the amount of bibenzyl, the decrement of DBDS at the temperature can be estimated. Furthermore, no difference due to the initial concentration was observed between the cases where the initial DBDS concentrations were 30 ppm and 300 ppm, respectively. This result shows that even with a change in the initial DBDS concentration, the relational expression between the amount of produced bibenzyl and the decrement of DBDS holds in the same way.

As such, it is understood that once the amount of produced bibenzyl and the temperature are determined, an estimated value of the decrement of DBDS can be obtained from a relational expression created in advance. Then, the sum of the quantitative value of the concentration of DBDS remaining in the transformer oil and the estimated value of the decrement of DBDS can provide an estimated value of the initial DBDS concentration.

Likewise, measurement of the dibenzyl sulfide concentration in the insulating oil (i.e., the amount of produced DBS) can provide an estimated value of the initial DBDS concentration. Fig. 2 shows a relationship between the dibenzyl sulfide concentration (in ppm) and the decrement of DBDS concentration (in ppm). Fig. 2 shows data sets of cases where the initial DBDS concentrations were 30 ppm and 300 ppm, respectively. In the drawing, "linear (300 ppm)" indicates a straight line fitted by the method of least square to the data set when the initial DBDS concentration was 300 ppm, the line being represented by the following relational expression: y = 23.08x, where y is a decrement of DBDS and x is a DBS concentration. As shown in Fig. 2, the amount of produced dibenzyl sulfide and the decrement of DBDS are correlated. Thus, from the amount of dibenzyl sulfide, the decrement of DBDS at the temperature can be estimated.

As such, it is understood that in the same manner as with bibenzyl, once the amount of dibenzyl sulfide and the temperature are determined, an estimated value of the decrement of DBDS can be obtained from a relational expression created in advance. Then, the sum of the quantitative value of the concentration of DBDS remaining in the transformer oil and the estimated value of the decrement of DBDS can provide an estimated value of the initial DBDS concentration.

As already described, the relationship between a by-product (here and hereinafter meaning bibenzyl and/or dibenzyl sulfide) and the decrement of DBDS is a function of temperature. Therefore, once the temperature dependency of this relationship is learned in advance, the relationship between the amount of produced by-product and DBDS at a given temperature can be obtained.

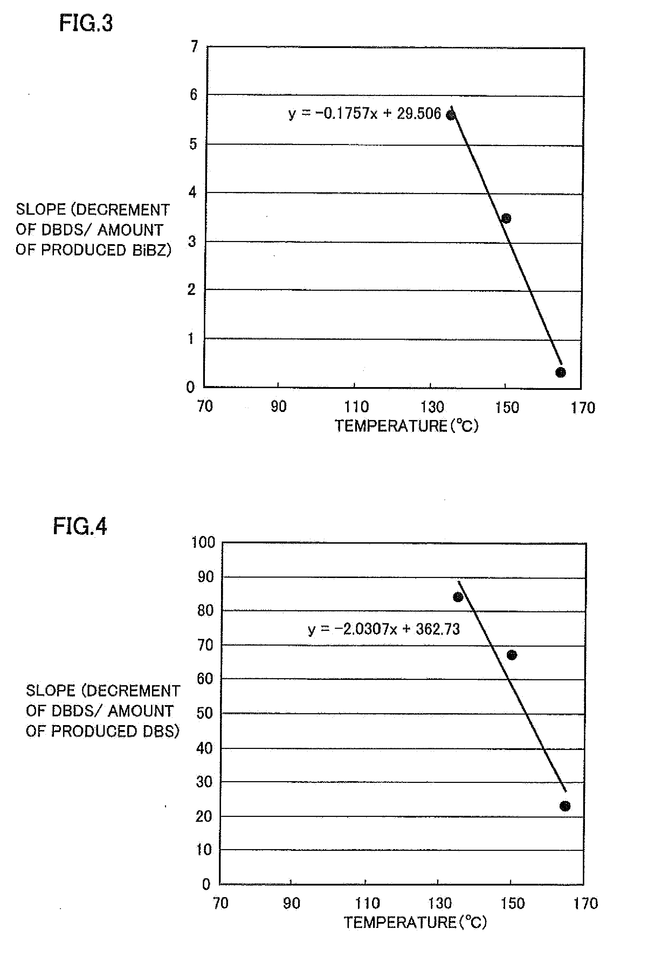

Fig. 3 is a graph showing a relationship between the temperature and a slope of the plotted relationship between the amount of bibenzyl and the decrement of DBDS (ratio by weight of decrement of DBDS/ amount of produced BiBZ). Note that the line segment in the drawing is a straight line fitted by the method of least square to a data set of the slope at each temperature, the line being represented by the following relational expression: y = 0.1757x + 29.506, where y is slope and x is temperature. As shown in Fig. 3, the slope and the temperature have a good linear relationship, which allows for calculation of a slope at a given temperature. Also, an amount of the by-product contained in an aged transformer oil and a residual amount of DBDS are quantified. A consumed amount of DBDS is then calculated from the product of the above-described slope and the amount of produced by-product. The sum of the consumed amount and the residual amount of DBDS is then calculated, and an estimated value of the initial DBDS concentration can thereby be obtained.

In addition, with respect to the amount of dibenzyl sulfide and the decrement of DBDS, the slope and the temperature also have a good linear relationship, as shown in Fig. 4. Thus, from the quantitative value (residual amount) of DBDS and the quantitative value of dibenzyl sulfide (the amount of the by-product), an estimated value of the initial DBDS concentration can be obtained. Further, for example, when the total amount of the equivalent amount of bibenzyl in mol and the equivalent amount of dibenzyl sulfide in mol is well correlated to the decrement of DBDS, it is also possible to obtain an estimated amount of the initial concentration of DBDS from the quantitative values of bibenzyl and dibenzyl sulfide.

As described above, the initial amount of DBDS in an insulating oil is proportional to the amount of produced copper sulfide, while a threshold value of the initial amount of DBDS varies depending on the structure, material and the like of an oil-filled electrical apparatus. For example, when an insulating material has an enough thickness, a larger acceptable value for an amount of produced copper sulfide is provided, and therefore, a higher threshold value is set. Conversely, when the insulator has a small thickness, a lower threshold value is set.

Examples of a specific method for setting the threshold value include threshold value determination methods using a test which is widely used as test for corrosive sulfur in insulating oils. Examples of this type of tests include JIS C 2101-17 (corrosive sulfur test) which is a test method specified by the Japanese Industrial Standards (JIS) and often used in Japan and ASTM D 1275B which is a test method of the ASTM (American Society for Testing and Materials) and often used outside Japan.

For example, in accordance with JIS C 2101-17 (corrosive sulfur test), a threshold value can be determined in the following procedures. First, an insulating oil exhibiting no corrosiveness under JIS C 2101-17 is prepared. For example, a sulfur-free synthetic oil such as alkyl benzene and α-olefin is preferable. A predetermined amount of DBDS is dissolved in this insulating oil to provide a sample oil. The sample oil prepared in this way is subjected to a test in a method as described in JIS C 2101-17.2 to 17.5 for determination of its corrosiveness.

For instance, in a case where sample oils in which DBDS of 50, 100, 150, and 200 ppm are dissolved, respectively, are determined as to their corrosiveness by the method above, if, for example, sample oils containing DBDS of 50 and 100 ppm, respectively exhibit noncorrosiveness and sample oils containing DBDS of 150 and 200 ppm exhibit corrosiveness, then 100 ppm which is the upper limit where noncorrosiveness was observed is set as a threshold value. When the initial DBDS concentration in an insulating oil of an oil-filled electrical apparatus exceeds this threshold value, a countermeasure can be taken, such as alerting that the oil-filled electrical apparatus is likely to experience a defective condition due to copper sulfide.

It should be construed that embodiment disclosed herein are by way of illustration in every respect, not by way of limitation. It is intended that the scope of the present invention is defined by claims, not by the above description, and includes all modifications and variations equivalent in meaning and scope to the claims.

## Claims

1. A prediction method for predicting the possibility of occurrence of anomaly in an oil-filled electrical apparatus, comprising compositionally analyzing an insulating oil collected from a transformer in operation, calculating an estimated value of a concentration of dibenzyl disulfide in said insulating oil in its virgin state from the compositional analysis, and predicting the possibility of occurrence of anomaly in an oil-filled electrical apparatus from a level or said estimated value.

2. The prediction method according to claim 1, wherein
concentrations of bibenzyl and/or dibenzyl sulfide and dibenzyl disulfide in said insulating oil are quantified by the compositional analysis of said insulating oil.

3. The prediction method according to claim 2, wherein
an estimated value of a decrement of the concentration of dibenzyl disulfide in said insulating oil relative to its virgin state is calculated from the concentration of bibenzyl and/or dibenzyl sulfide in said insulating oil quantified by the compositional analysis of said insulating oil,
said estimated value of the decrement is added to the concentration of dibenzyl disulfide in said insulating oil quantified by the compositional analysis, and
the estimated value of the concentration of dibenzyl disulfide in said insulating oil in its virgin state is calculated by the addition.

4. The prediction method according to claim 3, wherein
a relational expression between a decrement of dibenzyl disulfide and an amount of produced bibenzyl and/or dibenzyl sulfide at each temperature is created in advance by quantifying, with varied elapsed time periods and temperature conditions, a decrement of dibenzyl disulfide and an amount or produced dibenzyl and/or dibenzyl sulfide in an insulating oil to which dibenzyl disulfide has been added and which is after expiration of a predetermined time period, and
the estimated value of the decrement of the concentration of dibenzyl disulfide in said insulating oil relative to its virgin state is calculated based on said relational expression and from a temperature condition of said insulating oil and the concentration of bibenzyl and/or dibenzyl sulfide in said insulating oil quantified by the compositional analysis.

5. The prediction method according to claim 1, wherein
the concentration of dibenzyl disulfide in said insulating oil is quantified using gas chromatography.

6. The prediction method according to claim 1, wherein
the concentration of bibenzyl and/or dibenzyl sulfide in said insulating oil is quantified using gas chromatography.
